(19) Europäisches Patentamt
European Patent Office
Office européen des brevets

(11) Veröffentlichungsnummer: **0 310 015 A2**

(12) **EUROPÄISCHE PATENTANMELDUNG**

(21) Anmeldenummer: **88115958.6**

(51) Int. Cl.⁴: **C07K 5/02 , A61K 37/64**

(22) Anmeldetag: **28.09.88**

Patentansprüche für folgende Vertragsstaaten: ES + GR.

(30) Priorität: **30.09.87 DE 3732971**

(43) Veröffentlichungstag der Anmeldung: **05.04.89 Patentblatt 89/14**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Wagner, Adalbert, Dr.**
**Kleiststrasse 9**
**D-6238 Hofheim am Taunus(DE)**
Erfinder: **Kleeman, Heinz-Werner, Dr.**
**Jahnstrasse 6**
**D-6092 Kelsterbach(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim (Taunus)(DE)**
Erfinder: **Urbach, Hansjörg, Dr.**
**Le Lavandoustrasse 41**
**D-6242 Kronberg/Taunus(DE)**

(54) **Renin-hemmende Dipeptide, Verfahren zu deren Herstellung, diese enthaltende Mittel und ihre Verwendung.**

(57) Die Erfindung betrifft Verbindungen der Formel

$$R^1\text{-A-B-HN-}\overset{\displaystyle R^2}{\overset{|}{C}}\text{H-}\overset{\displaystyle R^9}{\overset{|}{C}}\text{H-}\overset{\displaystyle R^3}{\overset{|}{C}}\text{H-}R^4$$

worin $R^1$ abwesend ist oder Wasserstoff, Alkyl oder Acyl, A einen Acylrest oder einen Aminosäurerest, B einen Aminosäurerest bedeuten, $R^2$, $R^3$ und $R^4$ wie in der Beschreibung definiert sind und $R^9$ für -OH oder -F steht, sowie deren Salze, Verfahren zu ihrer Herstellung, diese enthaltende pharmazeutische Präparate und deren Verwendung als Arzneimittel sowie Zwischenprodukte zur Herstellung dieser Verbindungen.

EP 0 310 015 A2

# Renin-hemmende Dipeptide, Verfahren zu deren Herstellung, diese enthaltende Mittel und ihre Verwendung

Aus den EP-A-189 203, EP-A-230 266, EP-A-172 346, EP-A-172 347 und EP-A-229 667 sind Dipeptidderivate und deren Verwendung als Renin-Inhibitoren bekannt.

Es wurden neue Peptidderivate gefunden, die in vitro und in vivo hochwirksam das Enzym Renin hemmen.

Die Erfindung betrifft Verbindungen der Formel I

$$R^1\text{-A-B-HN-}\overset{\displaystyle R^2}{\overset{|}{C}}\text{H-}\overset{\displaystyle R^9}{\overset{|}{C}}\text{H-}\overset{\displaystyle R^3}{\overset{|}{C}}\text{H-}R^4 \qquad (I)$$

in welcher

$R^1$

$a_1$) abwesend ist oder Wasserstoff bedeutet,

$a_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,

($C_3$-$C_8$)-Cycloalkyl, ($C_3$-$C_8$)-Cycloalkyl-($C_1$-$C_{10}$)alkyl oder ($C_6$-$C_{14}$)-Aryl-($C_1$-$C_8$)-alkyl, das im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$a_3$) einen Rest der Formel II bedeutet,

$R^a$-W- (II)

worin W für -CO-, -O-CO-, -$SO_2$- oder -NH-CO- steht und $R^a$ für Wasserstoff, ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,

($C_3$-$C_8$)-Cycloalkyl,

($C_3$-$C_8$)-Cycloalkyl-($C_1$-$C_6$)-alkyl, ($C_6$-$C_{14}$)-Aryl, das gegebenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist, ($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S-oder O-Atom auch als Ringglieder steht, der gegebenenfalls wie ($C_6$-$C_{14}$)-Aryl unter $a_2$) definiert mono-, di- oder trisubstituiert ist,

A

$b_1$) einen Rest der Formel III oder IIIa bedeutet,

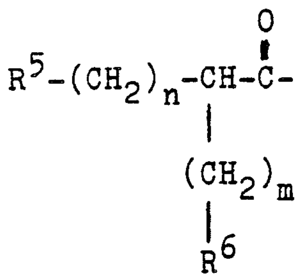

(III)

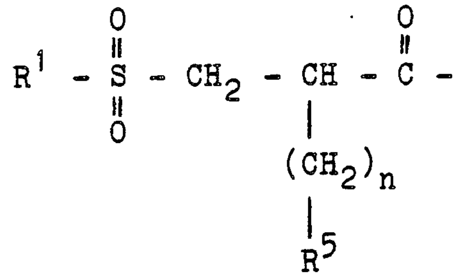

(IIIa)

in welchen
$R^1$ wie oben definiert ist, n und m gleich oder verschieden sind und 0, 1, 2, 3 oder 4 bedeuten, $R^5$ und $R^6$ gleich oder verschieden sind und Phenyl,
2- oder 3-Thienyl, 2-, 3-, oder 4-Pyridyl, 1-, 2- oder 4-Imidazolyl, 1- oder 2-Naphthyl, 2- oder 3-Benzo[b]-thienyl, OH oder Wasserstoff bedeuten oder

$b_2$) einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]-thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($b_2$) definiert, bedeutet,

$R^2$ Wasserstoff, $(C_1-C_{10})$-Alkyl, $(C_4-C_7)$-Cycloalkyl, $(C_4-C_7)$-Cycloalkyl-$(C_1-C_4)$-alkyl, $(C_6-C_{14})$-Aryl oder $(C_6-C_{14})$-Aryl-$(C_1-C_4)$-alkyl bedeutet,

$R^3$ Wasserstoff, $(C_1-C_{10})$-Alkyl, $(C_6-C_{14})$-Aryl oder $(C_6-C_{14})$-Aryl-$(C_1-C_4)$-alkyl bedeutet,

$R^4$ einen Rest der Formel IV bedeutet

$$-(CH_2)_p-X-(CH_2)_q-R^7 \quad (IV)$$

wobei X für $-CF_2-$, -CO- oder $-CHR^8-$ steht,
p und q unabhängig voneinander 0, 1, 2, 3 oder 4 bedeuten,
und

$R^8$ $(C_1-C_7)$-Alkyl, $(C_1-C_5)$-Alkoxy, $(C_1-C_5)$-Alkylthio, $(C_1-C_5)$-Alkylamino, -OH, $-N_3$, -F, -Cl, -Br oder -I bedeutet,

$R^7$ Wasserstoff, -OH, $-NH_2$, $(C_6-C_{14})$-Aryl oder Heteroaryl, das auch teilweise oder vollständig hydriert sein kann bedeutet und

$R^9$ -OH oder -F sein kann

sowie deren physiologisch verträgliche Salze.

Die durch $R^2$, $R^3$, $R^8$ und $R^9$ substituierten C-Atome können jeweils die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit $(C_1-C_6)$-Alkyl verknüpften unsubstituierten oder substituierten $(C_6-C_{14})$-Aryl-Rest, wie z.B. Benzyl, α- und β-Naphthylmethyl, Halobenzyl und Alkoxybenzyl, wobei Aralkyl jedoch nicht auf die genannten Reste beschränkt ist.

Unter einem Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom als Ringglieder werden Reste von Heteroaromaten verstanden, wie sie beispielsweise in Katritzky, Lagowski, Chemie der

Heterocyclen, Berlin, Heidelberg 1968, definiert sind. Der Heteroaromaten-Rest kann durch einen, zwei oder drei, vorzugsweise einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, Heteroaryloxy, Heteroarylthio und Heteroaryl-alkyl.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in denen die Reste wie folgt definiert sind:

$R^1$ ist vorzugsweise abwesend, bedeutet Wasserstoff oder steht für ($C_1$-$C_{10}$)-Alkyl, Cyclopentyl, Cyclohexyl, Cyclopentyl-($C_1$-$C_{10}$)-Alkyl, Cyclohexyl-($C_1$-$C_{10}$)-alkyl, gegebenenfalls substituiertes Phenyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, Carboxy-($C_1$-$C_{10}$)-alkyl, wie 2-Hydroxypropionyl oder 2-Hydroxy-3-methyl-butyryl, ($C_1$-$C_8$)-Alkanoyloxy-($C_1$-$C_{10}$)-alkyl, ($C_1$-$C_{11}$)-Alkanoyl, wie n-Decanoyl, Formyl, Acetyl, Pivaloyl, Isovaleryl oder Isobutyryl, gegebenenfalls geschütztes Amino-($C_1$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl; Di-($C_1$-$C_7$)-alkylamino-($C_2$-$C_{11}$)-alkanoyl, wie Dimethylaminoacetyl; ($C_4$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl; ($C_1$-$C_{10}$)-Alkoxycarbonyl, wie Methoxycarbonyl, Ethoxycarbonyl oder tert.-Butoxycarbonyl; durch Halogen substituiertes ($C_1$-$C_{10}$)-Alkoxycarbonyl, wie 2,2,2-Trichlorethoxycarbonyl oder 1,1-Dimethyl-2,2,2-trichlorethoxycarbonyl; ($C_6$-$C_{14}$)-Aryl-($C_1$-$C_6$)-alkoxycarbonyl, wie Benzyloxycarbonyl oder 9-Fluorenylmethoxycarbonyl;

$R^2$ ist vorzugsweise Isobutyl, Benzyl oder Cyclohexylmethyl,

$R^3$ ist vorzugsweise Wasserstoff, Isopropyl oder Isobutyl,

$R^4$, $R^8$ und $R^9$ sind vorzugsweise wie oben definiert,

$R^5$ und $R^6$ sind gleich oder verschieden und stehen vorzugsweise für Phenyl, 2-Thienyl, 2-Pyridyl, 1-Naphthyl, 2-Benzo[b]-thienyl, 3-Benzo[b]thienyl, OH oder Wasserstoff und

$R^7$ steht vorzugsweise für ein gegebenenfalls substituiertes Heteroaryl, das wie oben definiert ist, mit 1 oder 2 N-Atomen als Ringglieder;

n , p und q stehen bevorzugt für 0 oder 1.

Besonders bevorzugt sind Verbindungen der Formel I,
in denen

$R^1$ für Isobutylcarbonyl steht,

A Phe oder einen Rest der Formel IIIa bedeutet, worin $R^1$ ($C_1$-$C_6$)-Alkyl, das gegebenenfalls durch -$NH_2$ oder -COOH substituiert sein kann, ist; n 1 bedeutet und $R^5$ Phenyl, 2- oder 3-Thienyl ist,

B Histidin bedeutet,

$R^2$ Cyclohexylmethyl ist,

$R^3$ Wasserstoff ist,

$R^4$ für einen Rest der Formel IV steht, worin q 0 ist, p 1 oder 2 bedeutet, X -$CF_2$-, -CO- oder -$CHR^8$- ist und $R^8$ OH, F, Cl, Br, ($C_1$-$C_4$)-Alkoxy oder ($C_1$-$C_4$)-Alkyl, insbesondere F, bedeutet und

$R^9$ für OH steht

sowie deren physiologisch verträgliche Salze.

Bevorzugte Aminosäuren, die für die Reste A und B in Frage kommen, sind Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin. Daneben bedeutet A vorzugsweise einen Rest der Formel III wie unter ($b_1$) beschrieben.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln Va - Vc:

$R^1$-OH (Va)
$R^1$-A-OH (Vb)
$R^1$-A-B-OH (Vc)

Fragmente einer Verbindung der Formel I mit einer endständigen Aminogruppe besitzen die nachstehenden Formeln VIa - VIc:

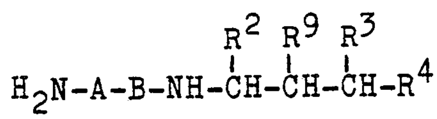
(VIa)

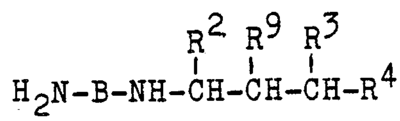
(VIb)

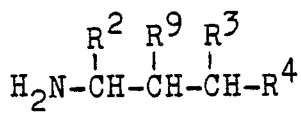
(VIc)

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:
Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel VII

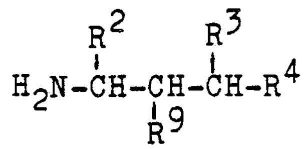
(VII)

worin $R^2$, $R^3$, $R^4$ und $R^9$ wie oben definiert sind, erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppe Aminoalkohole der

Formel VII ($R^9$ = OH) ergibt. Alternativ dazu werden in an sich bekannter Weise aus den geschützten Aminoaldehyden über die Allylamine die Epoxide hergestellt. Diese können entweder direkt mit den entsprechenden Arylalkyl-Nucleophilen umgesetzt werden, oder man öffnet das Epoxid zunächst mit Trimethylsilylchlorid und NaI in Acetonitril, spaltet den Silylether mit CsF und schützt das Iodid mit 2,2-Dimethoxypropan unter Säurekatalyse als Oxazolidin. Dieses Iodid kann mit weniger reaktiven Nucleophilen umgesetzt werden.

Zur Synthese der um eine $CH_2$-Gruppe verlängerten Arylalkyl-substituierten Aminoalkoholen geht man zum Beispiel von Boc-ACHPA-OEt (hergestellt nach J.Med.Chem. 28, 1779 (1985)) aus. Zunächst erfolgt N,O-Schützung, dann die Reduktion der Esterfunktion und schließlich die Umwandlung der Hydroxyfunktion in ein Bromid, das unter analogen Bedingungen wie die bereits genannten Elektrophile mit Arylalkyl-Nucleophilen umgesetzt werden kann. In Frage kommende Arylalkyl-Nucleophile sind z.B. Acetylimine und Acetylhydrazone. Weitere Verbindungen der Arylalkyl-substituierten Aminoalkohole mit verlängerter(n) $CH_2$-Gruppe(n) können nach den allgemein üblichen Methoden zur Kettenverlängerung erhalten werden.

Falls der gewählte Syntheseweg zu Diastereomeren bezüglich des $R^9$ tragenden Zentrums führt, können diese in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et al., J. org. Chem. 34, 2543 (1969)).

Die Herstellung N-geschützter Aminosäurealdehyde erfolgt nach B. Castro et al. (Synthesis 1983, 676).

Die Addition der Arylalkyl-Nucleophile an die genannten N-geschützten Elektrophile (bevorzugterweise N-tert.-Butoxycarbonyl- und Benzyloxycarbonyl Schutzgruppen) erfolgt in einem gegenüber Basen inerten Lösungsmittel wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäuren A oder B anfallen, können in an sich bekannter Weise durch fraktionierte Kristallisation oder durch Chromatographie getrennt werden.

Die Erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotension II spielt eine wesentliche Rolle bei der Blutdruckregulation, da es direkt den Blutdruck durch Gefäßkonstriktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, Schweine-Renin) gemessen. Hierzu wird z.B. Humanplasma, welches sowohl Renin als auch Angiotensinogen enthält, bei 37° C mit der zu testenden Verbindung inkubiert. Anschließend wird die Konzentration des während der Inkubation gebildeten Angiotensin I mit einem Radioimmunoassay gemessen. Die in der vorliegenden Erfindung beschriebenen Verbindungen der allgemeinen Formel I zeigen in verwendeten in-vitro-Tests Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt.

Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Zubereitungen, die diese Verbindungen enthalten sowie ein Verfahren zu deren Herstellung. Bevorzugt ist die Anwendung bei Primaten, insbesondere beim Menschen.

Pharmazeutische Präparate enthalten eine wirksame Menge des Wirkstoffs der Formel I zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

Verzeichnis der verwendeten Abkürzungen:

Ac Acetyl
ACHPA [3S,4S]-4-Amino-3-hydroxy-5-cyclohexylpentansäure
Boc tert.-Butoxycarbonyl
BuLi Butyl-Lithium
DAST Diethylaminoschwefeltrifluorid
DC Dünnschichtchromatographie
DCC Dicyclohexylcarbodiimid
DCI Desorption Chemical Ionisation
DIP Diisopropylether
DNP 2,4-Dinitrophenyl
DMF Dimethylformamid
DMSO Dimethylsulfoxid
EE Essigsäureethylester
EI Electron Impact
Etoc Ethoxycarbonyl
FAB Fast atom bombardment
H Hexan
HOBt 1-Hydroxybenzotriazol
Iva Isovaleryl
M Molekularpeak
MeOH Methanol
MS Massenspektrum
MTB Methyl-tert.-butylether
R.T. Raumtemperatur
Schmp. Schmelzpunkt
Sta [3S,4S]-4-Amino-3-hydroxy-6-methyl-heptansäure

Thi β-2-Thienylalanin
THF Tetrahydrofuran
Z Benzyloxycarbonyl

Die sonstigen für Aminosäuren verwendeten Abkürzungen entsprechen dem in der Peptidchemie üblichen drei Buchstaben-Code wie er z.B. in Europ. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

Iva-Phe-His-1(S)-cyclohexylmethyl-2(S)-hydroxy-6-oxo-6-phenyl-hexylamid

150 mg Iva-Phe-His(DNP)-1(S)-cyclohexylmethyl-2(S)-hydroxy-6-oxo-6-phenyl-hexylamid werden in 5 ml Acetonitril und 0,1 ml Thiophenol 10 h bei R.T. gerührt. Man engt im Vakuum ein und chromatographiert an Kieselgel (Laufmittel $CH_2Cl_2$/MeOH 12/1). Einengen der produkthaltigen Fraktionen liefert das Produkt als blaßgelbes Pulver.
$R_f$ ($CH_2Cl_2$/MeOH 12/1) = 0,2; MS (FAB) = 672 (M + 1)

a) Iva-Phe-His-(DNP)-1(S)-cyclohexylmethyl-2(S)-hydroxy-6-oxo-6-phenyl-hexylamid

Zu 300 mg Iva-Phe-His(DNP)-OH, 46 µl Pyridin und 80 µl N-Ethylpiperidin in 5 ml $CH_2Cl_2$ wird bei -5 °C 70 µl Pivaloylchlorid zugegeben. 30 Minuten wird bei + 5 °C bis + 10 °C gerührt, anschließend auf -10 °C gekühlt und 165 mg 1(S)-Cyclohexylmethyl-2(S)-hydroxy-6-oxo-6-phenyl-hexylamin in 5 ml $CH_2Cl_2$ zugetropft. Man rührt 16 h ohne Kühlung, engt im Vakuum ein und nimmt in 100 ml EE auf. Man wäscht 3 mal mit wäßriger $K_2CO_3$-Lösung, 1 mal mit $H_2O$, trocknet über $Na_2SO_4$ und engt im Vakuum ein. Nach Chromatographie an Kieselgel (Laufmittel EE) erhält man die Titelverbindung als schwach gelbes Pulver.
$R_f$ (EE) = 0,3; MS (FAB) = 838 (M + 1)

b) Iva-Phe-His(DNP)-OH

wurde nach der in J.Am.Chem.Soc. 86, 1839 (1964) beschriebenen Vorschrift hergestellt.

c) 1(S)-Cyclohexylmethyl-2(S)-hydroxy-6-oxo-6-phenyl-hexylamin

200 mg 3-Boc-4(S)-cyclohexylmethyl-2,2-dimethyl-5-(4-dimethylhydrazono-4-phenyl-butyl)-oxazolidin werden in 10 ml Dimethoxyethan gelöst, unter Kühlung (T ~ 10 °C) 10 ml gesättigte dimethoxyethanische HCl zugetropft und 3 h gerührt. Nun wird eingeengt und in $H_2O$ aufgenommen, mit gesättigter $Na_2CO_3$-Lösung auf pH 9-10 eingestellt und 3 mal mit EE extrahiert. Nach Trocknung erhält man die Titelverbindung, die ohne weitere Reinigung im nächsten Reaktionsschritt eingesetzt wird.

d) 3-Boc-4(S)-cyclohexylmethyl-2,2-dimethyl-5-(4-dimethylhydrazono-4-phenyl-butyl)-oxazolidin

Zu 203 mg Acetophenondimethylhydrazon (hergestellt nach der in J.Org.Chem. 31, 677 (1966) angegebenen Vorschrift) in 10 ml trockenem THF werden unter Argonatmosphäre 0,8 ml n-Butyl-Lithium (1,5 M in Hexan) bei -78 °C getropft. Nach 15 Minuten gibt man 303 mg 3-Boc-4(S)-cyclohexylmethyl-2,2-dimethyl-5-(2-bromethyl)-oxazolidin in 3 ml THF zu und rührt noch 2 h ohne Kühlung. Anschließend wird mit $H_2O$ versetzt und 3 mal mit EE extrahiert. Nach dem Trocknen über $Na_2SO_4$ wird eingeengt und an Kieselgel chromatographiert (Laufmittel: EE/Hexan 1/5).
$R_f$ (Hexan/EE 8/1) = 0,5; MS (DCI) = 487 (M + 1)

e) 3-Boc-4(S)-cyclohexylmethyl-2,2-dimethyl-5-(2-bromethyl)-oxazolidin

Zu 690 mg 3-Boc-4(S)-cyclohexylmethyl-2,2-dimethyl-5-(2-hydroxyethyl)-oxazolidin, 2,6 g Triphenylphosphin und 1,6 g Pyridiniumhydrobromid in 15 ml $CH_2Cl_2$ werden unter Argon 1,6 ml Azodicarbonsäurediethylester bei 20° C zugetropft. Nach 16 h bei R.T. wird $H_2O$ zugegeben und mit 100 ml $CH_2Cl_2$ verdünnt. Die organische Phase wird 2 mal mit gesättigter $NaHCO_3$-Lösung und 1 mal mit gesättigter NaCl-Lösung gewaschen. Die mit $Na_2SO_4$ getrocknete organische Phase wird eingeengt, der Rückstand in wenig EE aufgenommen und zur Abtrennung von $PPh_3$ filtriert. Reinigung an Kieselgel liefert die Titelverbindung (Laufmittel: H/EE 15/1).
$R_f$ (H/EE 15/1) = 0,3; MS (EI) 404 (M)

f) 3-Boc-4(S)-cyclohexylmethyl-2,2-dimethyl-5-(2-hydroxymethyl)-oxazolidin

10 g Boc-ACHPA-OEt (J.Med.Chem. 28, 1179 (1985)), 500 mg p-Toluolsulfonsäure und 7,2 ml Dimethoxypropan werden in 160 ml Toluol unter Argon 2 h auf 80° C erhitzt. Anschließend wird eingeengt. Der Rückstand wird bei 0° C zu einer Suspension aus 2 g $LiAlH_4$ in 200 ml THF zugetropft. Nach 2,5 h bei 0° C werden 100 ml 5%ige $NaHSO_4$-Lösung zugegeben und 3 mal mit EE extrahiert. Die vereinigten organischen Phasen werden 1 mal mit gesättigter $NaHCO_3$-Lösung gewaschen. Nach dem Trocknen mit $Na_2SO_4$ wird eingeengt und chromatographiert (Laufmittel: H/EE 2/1).
$R_f$ (H/EE 4/1) = 0,1; MS (EI) = 342 (M+1)

**Beispiel 2**

Iva-Phe-His-1(S)-cyclohexylmethyl-2(S)-hydroxy-6-oxo-6-(2-pyridyl)-hexylamid

Die Titelverbindung wurde aus Iva-Phe-His(DNP)-1(S)-cyclohexylmethyl-2(S)-hydroxy-6-oxo-6-(2-pyridyl)-hexylamid analog Beispiel 1 hergestellt.
$R_f$ (EE/MeOH 5/1) = 0,25; MS (FAB) = 673 (M+1)

a) Iva-Phe-His(DNP)-1(S)-cyclohexylmethyl-2(S)-hydroxy-6-oxo-6-(2-pyridyl)-hexylamid

270 mg 3-Boc-4(S)-cyclohexylmethyl-2,2-dimethyl-5-[4-(2-pyridyl)-4-oxobutyl]-oxazolidin werden in 5 ml Dimethoxyethan gelöst, unter Kühlung (T ~ 10° C) 5 ml gesättigte dimethoxyethanische HCl zugetropft und 3 h gerührt. Das Hydrochlorid erhält man durch Einengen bei R.T. Zur Kopplung wurden 335 mg Iva-Phe-His(DNP)-OH, 45 µl Pyridin und 230 µl N-Ethylpiperidin in 5 ml $CH_2Cl_2$ bei -5° C mit 68 µl Pivaloylchlorid versetzt. Nach 10 Minuten bei +10° C wird auf 0° C abgekühlt und das oben beschriebene Hydrochlorid in 2 ml $CH_2Cl_2$ zugetropft. Nach 2 h bei 0° C rührt man noch weitere 16 h bei R.T.. Aufarbeitung und Reinigung erfolgt analog Beispiel 1a).
$R_f$ (EE) = 0,1; MS (FAB) = 839 (M+1)

b) 3-Boc-4(S)-cyclohexylmethyl-2,2-dimethyl-5-[4-(2-pyridyl)-4-oxobutyl]-oxazolidin.

Zu 160 µl Diisopropylamin in 10 ml THF gibt man unter Argonatmosphäre bei -60° C 0,8 ml n-Butyl-Lithium (1,5 M in n-Hexan). Nach 1 h bei -50° C werden 225 mg N-[1-(2-pyridyl)ethyliden]cyclohexylamin in 3 ml THF zugegeben. Man läßt 1 h bei 0° C rühren, wobei eine intensive Rotfärbung auftritt. Nun gibt man 300 mg 3-Boc-4(S)-cyclohexylmethyl-2,2-dimethyl-5-(2-bromethyl)-oxazolidin in 2 ml THF zu und stoppt die Reaktion nach 1 h durch Zugabe von $H_2O$. Danach wird mit EE extrahiert und nach Trocknung über $Na_2SO_4$ und Einengen an Kieselgel chromatographiert (Laufmittel: Hexan/EE 4/1).
$R_f$ (Hexan/EE 4/1) = 0,3; MS (DCI) = 444 (M)

c) N-[1-(2-pyridyl)ethyliden]cyclohexylamin

20 g 2-Acetylpyridin, 30 g Cyclohexylamin und 0,5 g p-Toluolsulfonsäure werden in 250 ml Toluol unter Verwendung eines Wasserabscheiders 24 h zum Sieden erhitzt. Danach wird im Vakuum destilliert.
Sdp 0.05 torr = 102 °C

**Beispiel 3**

Iva-Phe-His-1(S)-cyclohexylmethyl-2(S),6(R,S)-dihydroxy-6-(2-pyridyl)-hexylamid

Die Titelverbindung wurde analog Beispiel 1 aus Iva-Phe-His(DNP)-1(S)-cyclohexylmethyl-2(S),6(R,S)-dihydroxy-6-(2-pyridyl)-hexylamid synthetisiert.
$R_f$ (EE/MeOH 3/1) = 0,6; MS (FAB) = 675 (M+1)

a) Iva-Phe-His(DNP)-1(S)-cyclohexylmethyl-2(S),6(R,S)-dihydroxy-6-(2-pyridyl)-hexylamid

Die Titelverbindung wurde aus 300 mg 3-Boc-4(S)-cyclohexylmethyl-2,2-dimethyl-5-[4(R,S)-hydroxy-4-(2-pyridyl)-butyl]-oxazolidin analog Beispiel 2a) erhalten.
$R_f$ (EE/MeOH 10/1) = 0,25 MS (FAB) = 841 (M+1)

b) 3-Boc-4(S)-cyclohexylmethyl-2,2-dimethyl-5-[4(R,S)-hydroxy-4-(2-pyridyl)-butyl]-oxazolidin

130 mg 3-Boc-4(S)-cyclohexylmethyl-2,2-dimethyl-5-[4-(2-pyridyl)-4-oxobutyl]-oxazolidin und 40 mg Natriumborhydrid werden in 5 ml Ethanol 90 Minuten bei R.T. gerührt. Nach Zugabe von 15 ml $H_2O$ wird 3mal mit EE extrahiert, über $Na_2SO_4$ getrocknet und eingeengt. Chromatographie an Kieselgel (Laufmittel EE/Hexan 1/1) liefert die Titelverbindung.
$R_f$ (EE/Hexan 1/1) = 0,4; MS (EI) = 446 (M)
Die Verbindungen der Beispiele 4 und 5 werden analog Beispiel 3 hergestellt.

**Beispiel 4**

Iva-Phe-His-1(S)-cyclohexylmethyl-2(R),6(R,S)-dihydroxy-6-(3-pyridyl)-hexylamid

$R_f$ (EE/MeOH 3/1) = 0,4; MS (FAB) = 675 (M+1)

**Beispiel 5**

Iva-Phe-His-1(S)-cyclohexylmethyl-2(R),6(R,S)-dihydroxy-6-(4-pyridyl)-hexylamid

$R_f$ (EE/Hexan 3/1) = 0,2; MS (FAB) = 675 (M+1)

**Beispiel 6**

Iva-Phe-His-1(S)-cyclohexylmethyl-2(S)-hydroxy-6(R,S)-methoxy-6-(2-pyridyl)-hexylamid

Die Titelverbindung wurde aus 3-Boc-4(S)-cyclohexylmethyl-2,2-dimethyl-5-[4(R,S)-methoxy-4-(2-pyridyl)-butyl]-oxazolidin analog Beispiel 2, 2a) erhalten.
$R_f$ (EE/MeOH 5/1) = 0,15; MS (FAB) = 689 (M+1).

a) 3-Boc-1(S)-cyclohexylmethyl-5-[4(R,S)-methoxy-4-(2-pyridyl)-butyl]-oxazolidin.

Die Titelverbindung wurde aus 3-Boc-4(S)-cyclohexylmethyl-2,2-dimethyl-5[4(R,S)-hydroxy-4-(2-pyridyl)-butyl]-oxazolidin analog Acta. Chem. Scand. 26, 1 (1972) erhalten.
$R_f$ (DIP) = 0,25; MS (FAB) = 461 (M+1)

**Beispiel 7**

Iva-Phe-His-1(S)-cyclohexylmethyl-2(S)-hydroxy-6(R,S)-ethoxy-6-(2-pyridyl)-hexylamid wurde analog Beispiel 6 erhalten.
$R_f$ (EE/MeOH 5/1) = 0,15; MS (FAB) = 703 (M+1)

**Beispiel 8**

Iva-Phe-His-1(S)-cyclohexylmethyl-2(S)-hydroxy-6(R,S)-fluoro-6-(4-pyridyl)-hexylamid

Die Titelverbindung wurde aus 3-Boc-4(S)-cyclohexylmethyl-2,2-dimethyl-5-[4(R,S)-fluoro-4-(4-pyridyl)-butyl]-oxazolidin analog Beispiel 2, 2a) erhalten.
$R_f$ (EE/MeOH 4/1) = 0,3; MS (FAB) = 677 (M+1)

a) 3-Boc-4(S)-cyclohexylmethyl-2,2-dimethyl-5-[4(R,S)-fluoro-4-(4-pyridyl)-butyl]-oxazolidin

160 mg 3-Boc-4(S)-cyclohexylmethyl-2,2-dimethyl-5-[4(R,S)-hydroxy-4-(4-pyridyl)-butyl]-oxazolidin in 2 ml $CH_2Cl_2$ werden zu einer Lösung von 50 µl Diethylaminoschwefeltrifluorid (DAST) unter Argonatmosphäre bei -35 °C zugetropft. Nach 75 Minuten gibt man 10 ml gesättigte $Na_2CO_3$-Lösung zu und extrahiert 3mal mit EE. Nach dem Trocknen über $Na_2SO_4$ und Einengen wird an Kieselgel chromatographiert (Laufmittel: Hexan/EE 1/1).
$R_f$ (Hexan/EE 1/1) = 0,3; MS (FAB) = 449 (M+1)

**Beispiel 9**

Iva-Phe-His-1(S)-cyclohexylmethyl-2(S),5(R,S)-dihydroxy-5-(2-pyridyl)-pentylamid

Die Titelverbindung wurde aus 4(S)-Cyclohexylmethyl-5-[3(R,S)-hydroxy-3-(2-pyridyl)-propyl]-oxazolidin-2-on analog Beispiel 2, 2a) synthetisiert.
$R_f$ (EE/MeOH 3/1) = 0,2; MS (FAB) = 661 (M+1)

a) 4(S)-cyclohexylmethyl-5-[3(R,S)-hydroxy-3-(2-pyridyl)-propyl]-oxazolidin-2-on

Die Titelverbindung wird aus 4(S)-Cyclohexylmethyl-5-[3-(2-pyridyl)-3-oxopropyl]-oxazolidin-2-on analog Beispiel 3 b) synthetisiert.
$R_f$ (EE) = 0,4; MS (FAB) = 319 (M+1)

b) 4(S)-cyclohexylmethyl-5-[3-(2-pyridyl)-3-oxopropyl]-oxazolidin-2-on

Zu 0,6 ml Diisopropylamin in 20 ml THF tropft man unter Argonatmosphäre bei -60 °C 3 ml n-Butyl-Lithium (1,5 M in Hexan) und rührt 45 Minuten bei 0 °C. Nun gibt man bei -50 °C 850 mg N-[1-(2-pyridyl)-ethyliden]cyclohexylamin in 3 ml THF zu und rührt dann 1 h bei 0 °C (Rotfärbung). Anschließend werden 380 mg Boc-2-cyclohexyl-1(S)-oxiran-2-(S)-yl-ethylamin in 5 ml THF zugetropft. Nach 60 Minuten bei 0 °C gibt man $H_2O$ zu und extrahiert 3mal mit EE. Nach Trocknen über $Na_2SO_4$ wird an Kieselgel chromatographiert (Laufmittel: Hexan/EE 1/1)
$R_f$ (Hexan/EE 1/1) = 0,3; MS (FAB) = 317 (M+1)

c) Boc-2-cyclohexylmethyl-1(S)-oxiran-2(S)-yl-ethylamin

2,6 g Boc-1(S)-cyclohexylmethyl-prop-2-en-1-yl-amin werden in 50 ml $CH_2Cl_2$ gelöst und 1,9 g m-Chlorperbenzoesäure in 50 ml $CH_2Cl_2$ bei 0° C zugetropft. 36 h wird bei R.T. gerührt, anschließend die Reaktionslösung zunächst mit 100 ml 5%iger wäßriger $Na_2SO_3$-Lösung, dann mit 100 ml gesättigter wäßriger $Na_2SO_3$-Lösung extrahiert. Nach Trocknung über $Na_2SO_4$ und Einengen im Vakuum wird an Kieselgel chromatographiert (Laufmittel: MTB/Cyclohexan 1/5) und man erhält die Titelverbindung als farbloses Öl, neben dem 2(R)-Isomeren.
$R_f$ (MTB/Cyclohexan 1/1) = 0,4; MS (FAB) = 270 (M+1)

d) Boc-1(S)-cyclohexylmethyl-prop-2-en-1-yl-amin

5,4 g Methyl-triphenylphosphoniumbromid werden in 100 ml THF suspendiert und bei R.T. 1,7 g Kalium-t-butylat unter Argon zugegeben. 2 h wird bei R.T. gerührt, auf 0° C abgekühlt und 3,9 g Boc-Cyclohexylalaninal in 50 ml THF zugetropft. 30 Minuten wird bei 0° C nachgerührt, 100 ml $H_2O$ zugetropft und das THF im Vakuum entfernt. 100 ml gesättigte wäßrige $NaHCO_3$-Lösung werden addiert und 3 mal mit 100 ml $CH_2Cl_2$ extrahiert. Nach Trocknung über $Na_2SO_4$ und Einengen im Vakuum wird an Kieselgel chromatographiert (Laufmittel: MTB/Cyclohexan 1/3) und man erhält die Titelverbindung als farbloses Öl.
$R_f$ (MTB/Cyclohexan 1/3) = 0,4; MS (DCI) = 254 (M+1)

e) Boc-cyclohexylalaninal

wurde nach der in J.Med.Chem. <u>28</u>, 1179 (1985) beschriebenen Methode erhalten.

**Beispiel 10**

Iva-Phe-His-1(S)-cyclohexylmethyl-2(S)-hydroxy-5(R,S)-fluoro-5-(2-pyridyl)-pentylamid

Die Titelverbindung wurde analog Beispiel 2, 2a) aus 3-Boc-4(S)-cyclohexylmethyl-2,2-dimethyl-5-[3-(R,S)-fluoro-3-(2-pyridyl)-propyl]-oxazolidin erhalten.
$R_f$ (EE/MeOH 5/1) = 0,3; MS (FAB) = 663 (M+1)

a) 3-Boc-4(S)-cyclohexylmethyl-2,2-dimethyl-5-[3(R,S)-fluoro-3-(2-pyridyl)-propyl]-oxazolidin

Die Titelverbindung wurde analog Beispiel 3 b) und 8 a) aus 3-Boc-4(S)-cyclohexylmethyl-2,2-dimethyl-5-[3-(2-pyridyl)-3-oxopropyl]-oxazolidin erhalten.
$R_f$ (EE/Hexan 1/1) = 0,7; MS (DCI) = 435 (M+1)

b) 3-Boc-4(S)-cyclohexylmethyl-2,2-dimethyl-5-[3-(2-pyridyl)-3-oxopropyl]-oxazolidin

Die Titelverbindung wurde analog Beispiel 9 b) aus 3-Boc-4(S)-cyclohexylmethyl-2,2-dimethyl-5(S)-iodmethyl-oxazolidin dargestellt.
$R_f$ (DIP) = 0,7; MS (EI) = 430 (M)

c) 3-Boc-4(S)-cyclohexylmethyl-2,2-dimethyl-5(S)-iodmethyl-oxazolidin

2,4 g Boc-2-cyclohexyl-1(S)-oxiran-2(S)-yl-ethylamin, 1.3 g NaI und 1.1 ml Trimethylsilylchlorid werden in 100 ml Acetonitril gelöst und 1,5 h bei R.T. gerührt. Anschließend wird 1 Äquivalent Cäsiumfluorid in THF und 5,3 ml Dimethoxyethan zugespritzt. 2,5 h wird bei R.T. gerührt, anschließend mit 200 ml gesättigter wäßriger $NaHCO_3$-Lösung versetzt und 3 mal mit 200 ml MTB extrahiert. Nach Trocknung über $Na_2SO_4$ und Einengen im Vakuum wird an Kieselgel chromatographiert (Laufmittel: MTB/Diisopropylether 1/2) und

man erhält die Titelverbindung als farbloses Öl.
$R_f$ (MTB/DIP 1/2) = 0,5; MS : 422 ($M-CH_3$)

**Beispiel 11**

Iva-Phe-His-1(S)-cyclohexylmethyl-2(S)-hydroxy-6(R,S)-fluoro-6-(2-pyridyl)-hexylamid

Die Titelverbindung wurde aus 3-Boc-4(S)-cyclohexylmethyl-2,2-dimethyl-5-[4(R,S)-fluoro-4-(2-pyridyl)-butyl]-oxazolidin analog Beispiel 2, 2 a) erhalten.
$R_f$ (EE/MeOH 5/1) = 0,2; MS (FAB) = 677 (M+1)

a) 3-Boc-4-(S)-cyclohexylmethyl-2,2-dimethyl-5-[4(R,S)-fluoro-4-(2-pyridyl)-butyl]-oxazolidin

wurde analog Beispiel 8 a) erhalten.
$R_f$ (H/EE 1/1) = 0,6; MS (FAB) = 449 (M+1)

**Beispiel 12**

Iva-Phe-His-1(S)-cyclohexylmethyl-2(S)-hydroxy-6,6-difluoro-6-(4-pyridyl)-hexylamid

Die Titelverbindung wurde analog Beispiel 2, 2 a) aus 3-Boc-4(S)-cyclohexylmethyl-2,2-dimethyl-5-[4,4-difluoro-4-(4-pyridyl)-butyl]-oxazolidin erhalten.
$R_f$ (EE/MeOH) = 0,2; MS (FAB) = 695 (M+1)

a) 3-Boc-4-(S)-cyclohexylmethyl-2,2-dimethyl-5-[4,4-difluoro-4-(4-pyridyl)-butyl]-oxazolidin

Zu 200 mg 3-Boc-4(S)-cyclohexylmethyl-2,2-dimethyl-5-[4-(4-pyridyl)-4-oxobutyl]-oxazolidin in 2 ml $CH_2Cl_2$ werden bei 0 °C 1 ml DAST zugegeben. Nach 2 Tagen bei R.T. werden 10 ml gesättigte $Na_2CO_3$-Lösung addiert und 3 mal mit EE extrahiert. Nach Trocknen mit $Na_2SO_4$ wird im Vakuum eingeengt und an Kieselgel chromatographiert (Eluens H/DIP 1,5/1), wobei die Titelverbindung als Öl anfällt.
$R_f$ (H/EE 1/1) = 0,45; MS = 467 (M+1)

**Beispiel 13**

Iva-Phe-His-1(S)-cyclohexylmethyl-2(S)-hydroxy-5,5-difluoro-5-(2-pyridyl)-pentylamid

Die Titelverbindung wurde aus 3-Boc-4(S)-cyclohexylmethyl-2,2-dimethyl-5-[3,3-difluoro-3-(2-pyridyl)-propyl]-oxazolidin analog Beispiel 12, 12 a) erhalten.
$R_f$ (EE/MeOH 5/1) = 0,3; MS (FAB) = 681 (M+1)

**Beispiel 14**

Iva-Phe-His-1(S)-cyclohexylmethyl-2(S)-hydroxy-5(R,S)-isopropyl-5-(2-pyridyl)-pentylamid

Die Titelverbindung wurde aus 3-Boc-4(S)-cyclohexylmethyl-2,2-dimethyl-5-[3(R,S)-isopropyl-3-(2-pyridyl)-propyl]-oxazolidin analog Beispiel 2, 2 a) erhalten.
$R_f$ (EE/MeOH 5/1) = 0,2; MS (FAB) = 687 (M+1)

a) 3-Boc-4(S)-cyclohexylmethyl-2,2-dimethyl-5-[3(R,S)-isopropyl-3-(2-pyridyl)-propyl]-oxazolidin

Zu 250 mg 2-Isobutylpyridin in 10 ml THF werden 1,3 ml 1,5 Mun-BuLi (Lösung in Hexan) bei -60°C addiert. Die Reaktionstemperatur wird dann 90 Minuten bei R.T. gehalten und nach abermaligem Abkühlen auf -60°C werden 500 mg 3-Boc-4(S)-cyclohexylmethyl-2,2-dimethyl-5-(2-bromethyl)-oxazolidin in 10 ml THF zugetropft. Nach 2 h bei -60°C werden 20 ml $H_2O$ zugegeben und das Rohprodukt durch 3-maliges Extrahieren mit EE isoliert. Chromatographie an Kieselgel (DIP/H 2/1) liefert das Produkt als Öl.
$R_f$ (DIP) = 0,6; MS (FAB) = 459 (M+1)

b) 2-Isobutylpyridin

Zu 5 g 2-Picolin in 50 ml Diethylether werden 40 ml 1,5 M n-BuLi (Lösung in Hexan) bei -60°C zugetropft. Nach 1,5 h bei R.T. wird abermals auf -60°C abgekühlt und 10 g 2-Iodpropan zugetropft. Nach 30 Minuten bei -60°C wird noch 60 Minuten bei R.T. gerührt. Danach werden 50 ml $H_2O$ zugegeben und das Rohmaterial durch 3-maliges Extrahieren mit Diethylether isoliert. Anschließend wird durch Destillation bei vermindertem Druck gereinigt. $Sdp._{40\ mm}$ = 85°C

**Beispiel 15**

Iva-Phe-His-1(S)-cyclohexylmethyl-2(S)-hydroxy-4-isopropyl-4-(2-pyridyl)-butylamid (Unpolares Isomere)

Die Titelverbindung wurde aus 3-Boc-4(S)-cyclohexylmethyl-2,2-dimethyl-5(S)-iodmethyl-oxazolidin analog Beispiel 14 erhalten.
$R_f$ (EE/MeOH 5/1) = 0,2; MS (FAB) = 673 (M+1)

**Beispiel 16**

Iva-Phe-His-1(S)-cyclohexylmethyl-2(S)-hydroxy-4-isopropyl-4-(2-pyridyl)-butylamid (Polares Isomere)

Die Titelverbindung wurde analog Beispiel 14 bzw. 15 erhalten.
$R_f$ (EE/MeOH 5/1 ) = 0,15; MS (FAB) = 673 (M+1)

**Beispiel 17**

Iva-Phe-His-1(S)-cyclohexylmethyl-2(S)-hydroxy-6(R,S)-ethoxy-6-(4-pyridyl)-hexylamid

Die Titelverbindung wurde analog Beispiel 6 erhalten.
$R_f$ (EE/MeOH 5/1 ) = 0,1; MS (FAB) = 703 (M+1)

**Beispiel 18**

Iva-Phe-His-1(S)-cyclohexylmethyl-2(S)-hydroxy-6(R,S)-n-propoxy-6-(4-pyridyl)-hexylamid

Die Titelverbindung wurde analog Beispiel 2, 2 a aus 3-Boc-4(S)-cyclohexylmethyl-2,2-dimethyl-5-[4-(R,S)-n-propoxy-4-(4-pyridyl)-butyl]-oxazolidin erhalten.
$R_f$ (EE/MeOH 5/1 ) = 0,15; MS (FAB) = 717 (M+1)

a) 3-Boc-4(S)-cyclohexylmethyl-2,2-dimethyl-5-[4(R,S)-n-propyl-4-(4-pyridyl)-butyl]-oxazolidin

Die Titelverbindung wurde analog Beispiel 6a mit n-Propylbromid als Alkylierungsreagenz erhalten.
$R_f$ (EE/Hexan 1/2) = 0,3; MS (DCI) = 489 (M + 1)

**Beispiel 19**

α) [3-t-Butylsulfonyl, 2(R)-phenylmethyl]propionyl-His-1(S)-cyclohexylmethyl-2(S)-hydroxy-5-(R,S)-fluor-5-(2-pyridyl)-pentylamid und

β) [3-t-Butylsulfonyl, 2(S)-phenylmethyl]propionyl-His-1(S)-cyclohexylmethyl-2(S)-hydroxy-5-(R,S)-fluor-5-(2-pyridyl)-pentylamid

Die beiden Titelverbindungen wurden aus 3-t-Butylsulfonyl, 2(R,S)-phenylmethyl-propionyl-His(DNP)-1-(S)-cyclohexylmethyl-2(S)-hydroxy-5(R,S)-fluor-5-(2-pyridyl)-pentylamid analog Beispiel 1 mit nachfolgender Trennung der Diastereomeren (α, β) an Kieselgel (Eluens:EE/MeOH 5/1) erhalten.

α) $R_f$ (EE/MeOH 5/1) = 0,25; MS (FAB) = 698 (M + 1)

β) $R_f$ (EE/MeOH 5/1) = 0,15; MS (FAB) = 698 (M + 1)

a) [3-t-Butylsulfonyl, 2(R,S)-phenylmethyl]propionyl-His(DNP)-1(S)-cyclohexylmethyl-2(S)-hydroxy-5-(R,S)-fluor 5-(2-pyridyl)-pentylamid

Zu 95 mg [3-t-Butylsulfonyl, 2(R,S)-phenylmethyl]propionsäure (s. Beispiel 22), 84 mg HOBt (85 %), 95 mg DCC in 10 ml THF wurden 200 mg H-His(DNP)-1(S)-cyclohexylmethyl-2-(S)hydroxy-5(R,S)-fluor-5-(2-pyridyl)-pentylamid in 5 ml THF bei 0 °C zugetropft. Danach rührt man 16 h bei RT. Nach Zugabe von 20 ml gesättigter $Na_2CO_3$ wurde 3 x mit 25 ml EE extrahiert, die vereinten organischen Phasen mit Natriumsulfat getrocknet und eingeengt. Das Gemisch wurde dann ohne weitere Reinigung im nächsten Reaktionsschritt verwendet.

b) H-His(DNP)-1-(S)-cyclohexylmethyl-2(S)-hydroxy-5-(R,S)-fluor-5-(2-pyridyl)-pentylamid

Die Titelverbindung wurde analog Beispiel 1c aus 300 mg Boc-His(DNP)-1(S)-cyclohexylmethyl-2(S)-hydroxy-5(R,S)-fluor-5-(2-pyridyl)-pentylamid erhalten und ohne weitere Reinigung verwendet.

c) Boc-His(DNP)-1(S)-cyclohexylmethyl-2-(S)-hydroxy-5-(R,S)-fluor-5-(2-pyridyl)-pentylamid

Die Titelverbindung wurde analog 1a aus Boc-His(DNP)-OH und Boc-1(S)-cyclohexylmethyl-2(S)-hydroxy-5(R,S)-fluor-5-(2-pyridyl)-pentylamid nach Abspaltung der Boc-Gruppe (1c) erhalten.
$R_f$ (EE/MeOH 10/1) = 0,5; MS (FAB) = 698 (M + 1)

d) Boc-1(S)-cyclohexylmethyl-2(S)-hydroxy-5(R,S)-fluor-5-(2-pyridyl)-pentylamid

Die Titelverbindung wurde analog Tetrahedron Lett. 28, 4185, (1987) bei 40 bis 50 °C aus 350 mg 3-Boc-4(S)- cyclohexylmethyl-5(S)-[3(R,S)-fluor-3-(2-pyridyl)-propyl]-oxazolidin-2-on erhalten.
$R_f$ (EE) = 0,55; MS (DCI) = 395 (M + 1)

e) 3-Boc-4(S)-cyclohexylmethyl-5(S)-[3(R,S)-fluor-3-(2-pyridyl)-propyl]-oxazolidin-2-on

300 mg 4-(S)-cyclohexylmethyl-5(S)-[3(R,S)-fluor-3-(2-pyridyl-propyl]-oxazolidin-2-on, 156 µl Triethylamin, 23 mg Dimethylaminopyridin und 265 mg Pyrokohlensäure-di-t-butylester wurden in 10 ml $CH_2Cl_2$ 16 h bei RT gerührt. Danach wurde eingeengt und an Kieselgel chromatographiert.
$R_f$ (EE) = 0,55; MS (DCI) = 421 (M + H)

f) 4-(S)-Cyclohexylmethyl-5(S)-[3(R,S)-fluor-3-(2-pyridyl)-propyl]-oxazolidin-2-on

Die Titelverbindung wurde aus 4(S)-cyclohexylmethyl-5(S)-jodmethyl-oxazolidin-2-on analog Beispiel 8a, 3b, 2b erhalten.
$R_f$ (EE) = 0,5; MS (DCI) = 321 ( M + 1)

g) 4(S)-Cyclohexylmethyl-5-(S)-jodmethyl-oxazolidin-2-on

Zu 45 g 3-p-Methoxybenzyl-4-(S)-cyclohexylmethyl-5(S)-jodmethyl-oxazolidin-2-on in 1500 ml Acetonitril/$H_2O$ 2/1 wurden 137 g Ammonium-cer-(IV)-nitrat bei RT portionsweise zugegeben. Nach 1 h wurde 3 x mit EE (500 ml) extrahiert, die vereinten organischen Phasen getrocknet ($Na_2SO_4$) und eingeengt. Chromatographie an Kieselgel (Eluens: Hexan/ EE 2/1) mit anschließender Kristallisation aus DIP lieferte die Titelverbindung.
$R_f$ (Hexan/EE 2/1) = 0,25; MS (DCI) = 324 (M + 1)

h) 3-p-Methoxybenzyl-4(S)-cyclohexylmethyl-5(S)-jodmethyl-oxazolidin-2-on

Zu 150 g $J_2$ in 1l $CH_2Cl_2$ wurden bei -50°C unter Argonatmosphäre 60 g N-p-Methoxybenzyl-N-Z-1(S)-cyclohexylmethyl-prop-2-en-1-yl-amin in 300 ml $CH_2Cl_2$ zugetropft. Nach 4 1/2 h bei dieser Temperatur wurden 1,5 l 5%ige $Na_2SO_3$ Lösung zugegeben und das Gemisch ohne Kühlung bis zur Entfärbung der Reaktionslösung geschüttelt. Nach einmaligem Waschen mit gesättigter NaCl-Lösung wurde getrocknet ($Na_2SO_4$) und eingeengt. Chromatographie an Kieselgel (Eluens: Hexan/EE 4/1) und anschließende Umkristallisation aus DIP lieferte die Titelverbindung.
$R_f$ (Hexan/EE 4/1) = 0,3; MS (DCI) = 444 (M + 1)

i) N-p-Methoxybenzyl-N-Z-1(S)-cyclohexylmethyl-prop-2-en-1-yl-amin

Zu 50 gN-Z-1(S)-cyclohexylmethyl-prop-2-en-1-yl-amin in 500 ml DMF wurden bei 0°C 9,4 g Natriumhydrid portionsweise zugegeben. Nach 2 h bei 0°C wurden 25 ml p-Methoxybenzylchlorid zugetropft. Nach 16 h bei RT wurden 100 ml 5% $NaHSO_4$ Lösung zugegeben und 3 x mit 300 ml EE extrahiert. Nach Trocknen mit $Na_2SO_4$ wurde eingeengt und an Kieselgel chromatographiert (Eluens: Hexan/EE 8/1).
$R_f$ (Hexan/EE 8/1) = 0,3; MS(DCI) = 408 (M + 1)

j) N-Z-1(S)-cyclohexylmethyl-prop-2-en-1-yl-amin

11 g 1(S)-Cyclohexylmethyl-prop-2-en-1-yl-amin und 12 g N-Z-Succinimid wurden in 200 ml DMF 16 h bei RT gerührt. Danach wurde eingeengt und an Kieselgel chromatographiert. (Eluens: Hexan/EE 4/1)
$R_f$ (Hexan/EE 2/1) = 0,55; MS (DCI) = 288 (M + 1)

k) 1(S)-Cyclohexylmethyl-prop-2-en-1-yl-amin

Die Titelverbindung wurde analog 1c aus Boc-1(S)-cyclohexylmethyl-prop-2-en-1-yl-amin erhalten.
Beispiele 20α und 20β wurden analog Beispiel 19 hergestellt.

**Beispiel 20**

α) [3-t-Butylsulfonyl, 2(R)-(2-thienylmethyl)]-propionyl-His-1(S)-cyclohexylmethyl-2(S)-hydroxy-5(R,S)-fluor-5-(2-pyridyl)-pentylamid
$R_f$ (EE/MeOH 5/1) = 0,25; MS (FAB) = 704 (M + 1)

β) [3-t-Butylsulfonyl, 2(S)-(2-thienylmethyl)]-propionyl-His-1(S)-cyclohexylmethyl-2(S)-hydroxy-5(R,S)-fluor-5-(2-pyridyl)-pentylamid.
$R_f$ (EE/MeOH 5/1) = 0,15; MS (FAB) = 704 (M + 1)

**Beispiel 21**

[3-t-Butylsulfonyl-2(R,S)-(2-thienylmethyl)]propionsäure

4,4 g [3-t-Butylsulfonyl-2(R,S)-(2-thienylmethyl)]-propionsäuremethylester werden in 50 ml 5N HCl suspendiert und 2 h zum Rückfluß erhitzt. Nach Abkühlung wird 3 x mit 50 ml EE extrahiert, über $Na_2SO_4$ getrocknet und das Solvens im Vakuum entfernt. Man erhält 4,0 g der Titelverbindung als blaßgelbes Öl. Die Auftrennung in das R und S-Isomere kann bei Bedarf analog EP 236 734 durchgeführt werden.
$R_f$ (MTB) = 0,15 - 0,25; MS (DCI): 291 (M+1)

a) [3-t-Butylsulfonyl,2(R,S)-(2-thienylmethyl)]propionsäuremethylester

8,4 g [3-t-Butylthio,2(R,S)-(2-thienylmethyl)]propionsäuremethylester werden in 100 ml $CH_2Cl_2$ gelöst und 10,6 g m-Chlorperbenzoesäure unter Eiskühlung portionsweise zugegeben. 1 h wird bei RT gerührt, anschließend zunächst mit 100 ml 10 % $Na_2SO_3$, dann mit 100 ml $NaHCO_3$ gewaschen. Über $Na_2SO_4$ wird getrocknet und das Solvens im Vakuum entfernt. Man erhält 7,2 g der Titelverbindung als farbloses Öl.
$R_f$ (MTB) = 0,56; MS (DCI): 305 (M+1)

b) [3-t-Butylthio,2(R,S)-(2-thienylmethyl)]propionsäuremethylester

6,1 ml t-Butylmercaptan werden in 100 ml MeOH (wasserfrei) gelöst und unter Argon 130 mg NaH zugegeben. Anschließend werden 7,6 g (2-(2-thienylmethyl)acrylsäuremethylester zugetropft und 4 h bei RT gerührt. Das Solvens wird im Vakuum entfernt, in 100 ml MTB aufgenommen und mit 100 ml 5 % $NaHSO_4$-Lösung gewaschen. Über $Na_2SO_4$ wird getrocknet und das Solvens im Vakuum entfernt. Man erhält 10,4 g der Titelverbindung als blaßgelbe Flüssigkeit, die ohne Reinigung und Charakterisierung weiter eingesetzt wird.
$R_f$ (DIP/H 1/5) = 0,31

c) 2-(2-Thienylmethyl)acrylsäure-methylester

11,8 g 2-Thienylmethylmalonsäure-monomethylester, 5,8 ml Diethylamin und 5,0 ml 36% wäßrige Formaldehyd-Lösung werden bei RT unter Argon 1 h gerührt. Das Wasser wird anschließend im Vakuum entfernt und der Rückstand chromatographiert. Man erhält 7,6 g der Titelverbindung als farblose Flüssigkeit.
$R_f$ (MTB/H 1/5) =. 0,49

d) 2-Thienylmethylmalonsäure-monomethylester

20,1 g 2-Thienylmethylmalonsäure-dimethylester werden in 300 ml MeOH gelöst und 5,0 g KOH zugegeben. Bei RT wird 7 h gerührt, das Lösungsmittel im Vakuum entfernt und mit je 100 ml 5 % $Na_2CO_3$-Lösung und EE aufgenommen. Anschließend wird die wäßrige Phase auf pH 2 angesäuert und 3 x mit je 100 ml EE extrahiert. Über $Na_2SO_4$ wird getrocknet und das Solvens im Vakuum entfernt. Man erhält 16,0 g der Titelverbindung als blaßgelbes Öl.
$R_f$ (EE/MeOH 6/1) = 0,3 - 0,4

e) 2-Thienylmethylmalonsäure-dimethylester

87,8 g Malonsäuredimethylester und 41,0 g Kalium-t-butylat werden unter Eiskühlung in 1,1 l THF

(wasserfrei) gelöst und unter Argon 44,1 g 2-Thienylmethylchlorid in 500 ml THF zugetropft. 3 h wird bei RT gerührt, das KCl abfiltriert, das Solvens im Vakuum entfernt und der Rückstand chromatographiert. Man erhält 33,8 g der Titelverbindung (I) als farbloses Öl neben 8,8 g Bis-(2-Thienylmethyl)-malonsäuredimethylester (II)
$R_f$ (I) (Toluol/DIP 20:1) = 0,35
$R_f$ (II) (Toluol/DIP 20:1) = 0,44

f) 2-Thienylmethylchlorid

252 g Thiophen werden in 128 ml konzentrierter wäßriger HCl suspendiert und bei 0°C 1h lang HCl-Gas eingeleitet. Anschließend werden ohne Unterbrechung des HCl-Stromes 255 ml 35% wäßrige Formaldehydlösung zugetropft und weitere 15 Minuten bei 0°C gerührt. Nach Abtrennung der organischen Phase wird die wäßrige Phase noch 2 x mit 600 ml $CH_2Cl_2$ extrahiert. Anschließend wird 2 x mit 600 ml gesättigter wäßriger $Na_2CO_3$ gewaschen, über $Na_2SO_4$ getrocknet, das Solvens im Vakuum entfernt und destilliert. Man erhält 174 g der Titelverbindung als farblose Flüssigkeit.
$Sdp._{22}$ = 81 - 84°C

**Beispiel 22**

[3-t-Butylsulfonyl,2(R,S)-phenylmethyl]propionsäure

Die Titelverbindung wurde analog EP 236 734 hergestellt. Die Auftrennung in der R- und S-Isomere erfolgte ebenfalls nach EP 236 734. Smp. 99 - 101°C; $R_f$(EE/H 1/1) = 0,16

**Beispiel 23**

Iva-Phe-His-1(S)-cyclohexylmethyl-2(S)-hydroxy-5(R,S)-ethoxy-5-(2-pyridyl)-pentylamid

Die Titelverbindung wurde aus Iva-Phe-His(DNP)-OH und 1(S)-Cyclohexylmethyl-2(S)-hydroxy-5(R,S)-ethoxy-5-(2-pyridyl)-pentylamin analog Beispiel 1, 1a und Beispiel 10 erhalten.
$R_f$ (EE/MeOH 5/1) = 0,15; MS(FAB) = 689 (M + 1)

**Ansprüche**

1. Verbindung der Formel I

$$R^1\text{-A-B-HN-}\overset{\displaystyle R^2}{\underset{|}{C}}\text{H-}\overset{\displaystyle R^9}{\underset{|}{C}}\text{H-}\overset{\displaystyle R^3}{\underset{|}{C}}\text{H-}R^4 \qquad (I)$$

in welcher

$R^1$
$a_1$) abwesend ist oder Wasserstoff bedeutet,
$a_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,
($C_3$-$C_8$)-Cycloalkyl, ($C_3$-$C_8$)-Cycloalkyl-($C_1$-$C_{10}$)alkyl oder ($C_6$-$C_{14}$)-Aryl-($C_1$-$C_8$)-alkyl, das im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$a_3$) einen Rest der Formel II bedeutet,

$R^a$-W- (II)

worin W für -CO-, -O-CO-, -SO$_2$- oder -NH-CO- steht und $R^a$ für Wasserstoff, ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist, ($C_3$-$C_8$)-Cycloalkyl, ($C_3$-$C_8$)-Cycloalkyl-($C_1$-$C_6$)-alkyl, ($C_6$-$C_{14}$)-Aryl, das gegebenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist,
($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S-gegebenenfalls wie ($C_6$-$C_{14}$)-Aryl unter $a_2$) definiert mono-, di- oder trisubstituiert ist,

A
$b_1$) einen Rest der Formel III oder IIIa bedeutet,

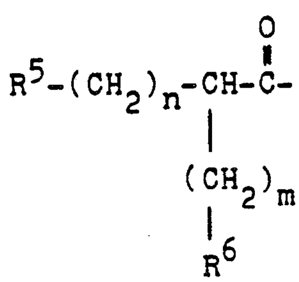

(III)

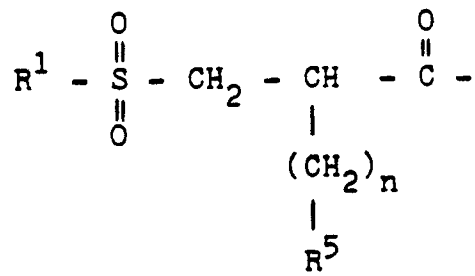

(IIIa)

in welcher
$R^1$ wie oben definiert ist, n und m gleich oder verschieden sind und 0, 1, 2, 3 oder 4 bedeuten,
$R^5$ und $R^6$ gleich oder verschieden sind und Phenyl, 2- oder 3-Thienyl, 2-, 3- oder 4-Pyridyl, 1-, 2-oder 4-Imidazolyl, 1- oder 2-Naphthyl, 2- oder 3-Benzo[b]thienyl, OH oder Wasserstoff bedeuten oder
$b_2$) einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]-thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-ylalanin, N-Methyl-Histidin, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($b_2$) definiert, bedeutet,
$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet,
$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,
$R^4$ einen Rest der Formel IV bedeutet

$-(CH_2)_p - X - (CH_2)_q - R^7$ (IV)

wobei X für $-CF_2-$, -CO- oder $-CHR^8-$ steht,
p und q unabhängig voneinander 0, 1, 2, 3 oder 4 bedeuten,
und
$R^8$ ($C_1$-$C_7$)-Alkyl, ($C_1$-$C_5$)-Alkoxy, ($C_1$-$C_5$)-Alkylthio, ($C_1$-$C_5$)-Alkylamino, -OH, $-N_3$, -F, -Cl, -Br oder -I bedeutet,
$R^7$ Wasserstoff, -OH, $-NH_2$, ($C_6$-$C_{14}$)-Aryl oder Heteroaryl, das auch teilweise oder vollständig hydriert sein kann bedeutet und
$R^9$ -OH oder -F sein kann
sowie deren physiologisch verträgliche Salze.

2. Verbindung der Formel I gemäß Anspruch 1, in welcher $R^1$ abwesend ist; Wasserstoff bedeutet oder für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl oder Carboxy-($C_1$-$C_{10}$)-alkyl steht, sowie deren physiologisch verträgliche Salze.

3. Verbindung der Formel I gemäß Anspruch 1, in welcher
$R^1$ ($C_1$-$C_{11}$)-Alkanoyl; gegebenenfalls geschütztes Amino-($C_1$-$C_{11}$)-alkanoyl; Di-($C_1$-$C_7$)-alkylamino-($C_2$-$C_{11}$)-alkanoyl; ($C_4$-$C_9$)-Cycloalkylcarbonyl; ($C_6$-$C_{10}$)-Aryl-($C_2$-$C_{11}$)-alkanoyl; gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl; Pyrrolyl-2-carbonyl; Pyridyl-3-carbonyl; Benzolsulfonyl; ($C_1$-$C_{10}$)-Alkoxycarbonyl; durch Halogen substituiertes ($C_1$-$C_{10}$)-Alkoxycarbonyl; oder ($C_6$-$C_{14}$)-Aryl-($C_1$-$C_6$)-alkoxycarbonyl bedeutet, sowie deren physiologisch verträgliche Salze.

4. Verbindung der Formel I gemäß einem oder mehreren der Ansprüche 1-3, in welcher
$R^2$ Isobutyl, Benzyl oder Cyclohexylmethyl bedeutet, sowie deren physiologisch verträgliche Salze.

5. Verbindung der Formel I gemäß einem oder mehreren der Ansprüche 1-4, in welcher
$R^3$ Wasserstoff, Isopropyl oder Isobutyl bedeutet, sowie deren physiologisch verträgliche Salze.

6. Verbindung der Formel I gemäß einem oder mehreren der Ansprüche 1-5, in welcher
$R^4$ und $R^8$ wie in Anspruch 1 definiert sind, sowie deren physiologisch verträgliche Salze.

7. Verbindung der Formel I gemäß einem oder mehreren der Ansprüche 1-6, in welcher
$R^5$ und $R^6$ gleich oder verschieden sind und Phenyl, 2-Thienyl, 2-Pyridyl, 1-Naphthyl, 2-Benzo[b]thienyl, OH oder Wasserstoff bedeuten, sowie deren physiologisch verträgliche Salze.

8. Verbindung der Formel I gemäß einem oder mehreren der Ansprüche 1-7, in welcher
$R^7$ für ein gegebenenfalls substituiertes Heteroaryl steht, das wie in Anspruch 1 definiert ist, jedoch 1 oder 2 N-Atome als Ringglieder enthält, sowie deren physiologisch verträgliche Salze.

9. Verbindung der Formel I gemäß einem oder mehreren der Ansprüche 1-8, in welcher A und B gleich oder verschieden sind und Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeuten, sowie deren physiologisch verträgliche Salze.

10. Verbindung der Formel I gemäß einem oder mehreren der Ansprüche 1-8, in welcher A einen Rest der Formel III bedeutet, der wie im Anspurch 1 definiert ist, sowie deren physiologisch verträgliche Salze.

11. Verbindung der Formel I gemäß einem oder mehreren der Ansprüche 1 bis 10, in welcher
$R^1$ für Isobutylcarbonyl steht,
A Phe oder einen Rest der Formel IIIa bedeutet,
worin $R^1$ ($C_1$-$C_6$)-Alkyl, das gegebenenfalls durch $-NH_2$ oder -COOH substituiert sein kann, ist,
n 1 bedeutet und $R^5$ Phenyl, 2- oder 3-Thienyl ist,
B Histidin bedeutet,
$R^2$ Cyclohexylmethyl ist,
$R^3$ Wasserstoff ist,
$R^4$ für einen Rest der Formel IV steht,
worin q 0 ist, p 1 oder 2 bedeutet, X $-CF_2-$, -CO-oder $-CHR^8$ ist und $R^8$ OH, F, Cl, Br, ($C_1$-$C_4$)-Alkoxy oder ($C_1$-$C_4$)-Alkyl, insbesondere F, bedeutet und
$R^9$ für OH steht
sowie deren physiologisch verträgliche Salze.

12. Verfahren zur Herstellung einer Verbindung der Formel I gemäß einem oder mehreren der Ansprüche 1 bis 11, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

13. Verwendung einer Verbindung gemäß einem oder mehreren der Ansprüche 1 bis 11 als Heilmittel.

14. Verwendung einer Verbindung gemäß einem oder mehreren der Ansprüche 1 bis 11 als Heilmittel bei der Behandlung des Bluthochdrucks.

15. Pharmazeutische Zubereitung enthaltend eine Verbindung gemäß einem oder mehreren der Ansprüche 1 bis 11 oder deren physiologisch verträgliches Salz.

16. Verfahren zur Herstellung einer Zubereitung gemäß Anspruch 15, dadurch gekennzeichnet, daß man eine Verbindung der Formel I oder deren physiologisch verträgliches Salz zusammen mit einem physiologisch annehmbaren Träger und gegebenenfalls weiteren Zusatz-, Hilfs- und/oder Konservierungsstoffen in eine geeignete Darreichungsform bringt.

Patentansprüche für folgende Vertragsstaaten : ES; GR

1. Verfahren zur Herstellung einer Verbindung der Formel I

$$R^1\text{-A-B-HN-}\underset{}{\overset{R^2}{\overset{|}{C}}}\text{H-}\overset{R^9}{\overset{|}{C}}\text{H-}\overset{R^3}{\overset{|}{C}}\text{H-}R^4 \qquad (I)$$

in welcher

$R^1$

$a_1$) abwesend ist oder Wasserstoff bedeutet,

$a_2$) ($C_1$-$C_{20}$)-Alkyl, das gegebenenfalls durch ein, zwei oder drei gleiche oder verschiedene Reste aus der Reihe Hydroxy, ($C_1$-$C_7$)-Alkoxy, Carboxy, ($C_1$-$C_7$)-Alkoxycarbonyl, ($C_1$-$C_8$)-Alkanoyloxy, Cl, Br, Amino, ($C_1$-$C_7$)-Alkylamino, Di-($C_1$-$C_7$)-alkylamino, ($C_1$-$C_5$)-Alkoxycarbonylamino oder ($C_7$-$C_{11}$)-Aralkyloxycarbonylamino substituiert ist,

($C_3$-$C_8$)-Cycloalkyl, ($C_3$-$C_8$)-Cycloalkyl-($C_1$-$C_{10}$)alkyl oder ($C_6$-$C_{14}$)-Aryl-($C_1$-$C_8$)-alkyl, das im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist, bedeutet oder

$a_3$) einen Rest der Formel II bedeutet,

$R^a$-W- (II)

worin W für -CO-, -O-CO-, -$SO_2$- oder -NH-CO- steht und $R^a$ Wasserstoff, ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,

($C_3$-$C_8$)-Cycloalkyl, ($C_3$-$C_8$)-Cycloalkyl-($C_1$-$C_6$)-alkyl, ($C_6$-$C_{14}$)-Aryl, das gegebenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist,

($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S-oder O-Atom auch als Ringglieder steht, der gegebenenfalls wie ($C_6$-$C_{14}$)-Aryl unter $a_2$) definiert mono-, di- oder trisubstituiert ist,

A

$b_1$) einen Rest der Formel III oder IIIa bedeutet,

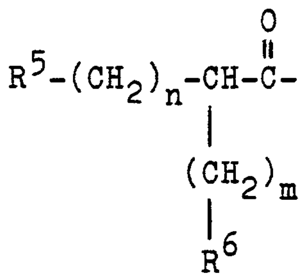

(III)

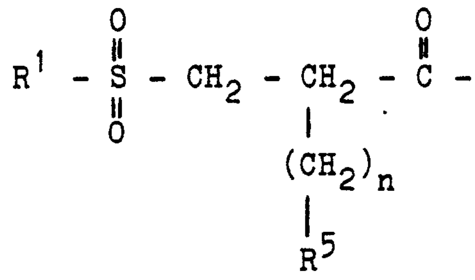

(IIIa)

in welcher

$R^1$ wie oben definiert ist, n und m gleich oder verschieden sind und 0, 1, 2, 3 oder 4 bedeuten;

$R^5$ und $R^6$ gleich oder verschieden sind und Phenyl, 2- oder 3-Thienyl, 2-, 3- oder 4-Pyridyl, 1-, 2-oder 4-Imidazolyl, 1- oder 2-Naphthyl, 2- oder 3-Benzo[b]thienyl, OH oder Wasserstoff bedeuten oder

$b_2$) einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo[b]-thienylalanin, $\beta$-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-ylalanin, N-Methyl-Histidin, 2-Amino-4-(3-thienyl)-buttersäure, 3-(2-Thienyl)-serin, (Z)-Dehydrophenylalanin und (E)-Dehydrophenylalanin bedeutet,

B einen Rest einer Aminosäure, wie unter ($b_2$) definiert, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet,

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

$R^4$ einen Rest der Formel IV bedeutet

$$-(CH_2)_p - X - (CH_2)_q - R^7 \quad (IV)$$

wobei X für $-CF_2-$, -CO- oder $-CHR^8-$ steht,

p und q unabhängig voneinander 0, 1, 2, 3 oder 4 bedeuten,

und

$R^8$ ($C_1$-$C_7$)-Alkyl, ($C_1$-$C_5$)-Alkoxy, ($C_1$-$C_5$)-Alkylthio, ($C_1$-$C_5$)-Alkylamino, -OH, $-N_3$, -F, -Cl, -Br oder -I bedeutet,

$R^7$ Wasserstoff, -OH, $-NH_2$, ($C_6$-$C_{14}$)-Aryl oder Heteroaryl, das auch teilweise oder vollständig hydriert sein kann bedeutet und

$R^9$ -OH oder -F sein kann

sowie deren physiologisch verträgliche Salze,

dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß man eine Verbindung der Formel I herstellt, in welcher $R^1$ abwesend ist, Wasserstoff bedeutet oder für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl oder Carboxy-($C_1$-$C_{10}$)-alkyl steht, sowie deren physiologisch verträgliche Salze.

3. Verfahren gemäß Anspruch 1, dadurch gekennzeichnet, daß man eine Verbindung der Formel I herstellt, in welcher $R^1$ ($C_1$-$C_{11}$)-Alkanoyl; gegebenenfalls geschütztes Amino-($C_1$-$C_{11}$)-alkanoyl; Di-($C_1$-$C_7$)-alkylamino-($C_2$-$C_{11}$)-alkanoyl; ($C_4$-$C_9$)-Cycloalkylcarbonyl; ($C_6$-$C_{10}$)-Aryl-($C_2$-$C_{11}$)-alkanoyl; gegebenenfalls durch Halogen, ($C_1$-$C_7$)-Alkyl, ($C_1$-$C_7$)-Alkoxy, ($C_1$-$C_7$)-Alkoxycarbonyl substituiertes Benzoyl; Pyrrolyl-2-carbonyl; Pyridyl-3-carbonyl; Benzolsulfonyl; $(C_1-C_{10})$-Alkoxycarbonyl; durch Halogen substituiertes $(C_1-C_{10})$-Alkoxycarbonyl; oder $(C_6-C_{14})$-Aryl-$(C_1-C_6)$-alkoxycarbonyl bedeutet, sowie deren physiologisch verträgliche Salze.

4. Verfahren gemäß einem oder mehreren der Ansprüche 1 bis 3, dadurch gekennzeichnet, daß man eine Verbindung der Formel I herstellt, in welcher $R^2$ Isobutyl, Benzyl oder Cyclohexylmethyl bedeutet, sowie deren physiologisch verträgliche Salze.

5. Verfahren gemäß einem oder mehreren der Ansprüche 1 bis 4, dadurch gekennzeichnet, daß man eine Verbindung der Formel I herstellt, in welcher $R^3$ Wasserstoff, Isopropyl oder Isobutyl bedeutet, sowie deren physiologisch verträgliche Salze.

6. Verfahren gemäß einem oder mehreren der Ansprüche 1 bis 5, dadurch gekennzeichnet, daß man eine Verbindung der Formel I herstellt, in welcher $R^4$ und $R^8$ wie in Anspruch 1 definiert sind, sowie deren physiologisch verträgliche Salze.

7. Verfahren gemäß einem oder mehreren der Ansprüche 1 bis 6, dadurch gekennzeichnet, daß man eine Verbindung der Formel I herstellt, in welcher $R^5$ und $R^6$ gleich oder verschieden sind und Phenyl, 2-Thienyl, 2-Pyridyl, 1-Naphthyl, 2-Benzo[b]thienyl, OH oder Wasserstoff bedeuten, sowie deren physiologisch verträgliche Salze.

8. Verfahren gemäß einem oder mehreren der Ansprüche 1 bis 7, dadurch gekennzeichnet, daß man eine Verbindung der Formel I herstellt, in welcher $R^7$ für ein gegebenenfalls substituiertes Heteroaryl steht, das wie in Anspruch 1 definiert ist, jedoch 1 oder 2 N-Atome als Ringglieder enthält, sowie deren physiologisch verträgliche Salze.

9. Verfahren gemäß einem oder mehreren der Ansprüche 1 bis 8, dadurch gekennzeichnet, daß man eine Verbindung der Formel I herstellt, in welcher A und B gleich oder verschieden sind und Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin oder (E)-Dehydrophenylalanin bedeuten, sowie deren physiologisch verträgliche Salze.

10. Verfahren gemäß einem oder mehreren der Ansprüche 1 bis 9, dadurch gekennzeichnet, daß man eine Verbindung der Formel I herstellt, in welcher A einen Rest der Formel III bedeutet, der wie im Anspruch 1 definiert ist, sowie deren physiologisch verträgliche Salze.

11. Verfahren gemäß einem oder mehreren der Ansprüche 1 bis 10, dadurch gekennzeichnet, daß man eine Verbindung der Formel I herstellt, in welcher

$R^1$ für Isobutylcarbonyl steht,

A Phe oder einen Rest der Formel IIIa bedeutet,

worin $R^1$ $(C_1-C_6)$-Alkyl, das gegebenenfalls durch $-NH_2$ oder -COOH substituiert sein kann, ist,

n 1 bedeutet und $R^5$ Phenyl, 2- oder 3-Thienyl ist,

B Histidin bedeutet,

$R^2$ Cyclohexylmethyl ist,

$R^3$ Wasserstoff ist,

$R^4$ für einen Rest der Formel IV steht,

worin q 0 ist, p 1 oder 2 bedeutet, X $-CF_2-$, -CO-oder $-CHR^8$ ist und $R^8$ OH, F, Cl, Br, $(C_1-C_4)$-Alkoxy oder $(C_1-C_4)$-Alkyl, insbesondere F, bedeutet und

$R^9$ für OH steht

sowie deren physiologisch verträgliche Salze.

12. Verfahren zur Herstellung eines pharmazeutischen Mittels, enthaltend eine Verbindung der Formel I gemäß einem oder mehreren der Ansprüche 1 bis 11, dadurch gekennzeichnet, daß man diese und einen Träger in eine geeignete Darreichungsform bringt.